# EUROPEAN PATENT SPECIFICATION

(11) **EP 1 221 990 B1**
(45) Date of publication and mention of the grant of the patent: **31.03.2010**
(21) Application number: 00966101.8
(22) Date of filing: 28.09.2000
(51) Int. Cl.: A61M 15/00, B65D 83/14

(54) **MEDICAMENT DISPENSER**
ABGABEVORRICHTUNG FÜR ARZNEIMITTEL
DISTRIBUTEUR DE MEDICAMENTS

(30) Priority: 21.10.1999 GB 9924808
(43) Date of publication of application: 17.07.2002
(73) Proprietor: GLAXO GROUP LIMITED, Greenford, Middlesex UB6 ONN (GB)
(72) Inventor: RAND, Paul Kenneth, Herts. SG12 0DP (GB)
(74) Representative: Rice, Jason Neale
(86) International application number: PCT/EP2000/009641
(87) International publication number: WO 2001/028608

(56) References cited:
- WO-A-99/47195
- WO-A-99/47195
- WO-A1-95/30607
- WO-A2-96/08284
- DE-A1- 3 115 568
- GB-A- 997 088
- GB-A- 997 088
- GB-A- 2 129 322
- US-A- 2 877 994
- US-A- 3 971 377
- US-A- 4 184 778
- US-A- 4 184 778
- US-A- 4 612 291
- US-A- 4 759 635
- US-A- 5 352 036
- US-A- 5 397 178
- US-A- 5 397 178
- US-A- 5 449 493
- US-A- 5 622 166
- PATENT ABSTRACTS OF JAPAN vol. 011, no. 054 (M-563), 19 February 1987 (1987-02-19) & JP 61 217431 A (KOBE STEEL LTD), 27 September 1986 (1986-09-27)
- PATENT ABSTRACTS OF JAPAN vol. 011, no. 054 (M-563), 19 February 1987 (1987-02-19) & JP 61 217431 A (KOBE STEEL LTD), 27 September 1986 (1986-09-27)

## Description

### Technical Field

This invention relates to a medicament dispenser including an agitator for agitating the medicament contents thereof. The dispenser is particularly suitable for use as an inhalation device.

### Background to the Invention

Known medicament dispensers comprise a medicament container having a dispensing outlet for dispensing of the medicament therefrom. Such medicament dispensers often require the patient to agitate the contents thereof prior to dispensing to ensure ready and uniform dispensing of the medicament. The agitation involves a manual shaking action.

Agitation is particularly required where the dispenser comprises medicament as a suspension in a propellant formulation. A well-known example of this type is the metered dose inhaler for dispensing of respiratory medicament which comprises an aerosol container having a dispensing valve. The medicament is comprised within the aerosol container in the form of a suspension comprising propellant and optionally other additives such as solvents or surfactants. Such suspensions have a tendency to settle out, sediment or cream. A pre-dispensing agitation step is necessary to re-establish a uniform suspension so that uniform dispensing of medicament may be achieved.

Agitation can also be required where the medicament is in powder form. It is known that powders can tend to settle out, agglomerate or even cake on storage. The agitation therefore performs the function of breaking up any agglomerates or cakes that may have formed, and thereby ensures that the powder is readily dispensable.

Various suggestions have been made to enhance manual shaking action to maximise the effect of agitation.

PCT Patent Application No. WO95/30607 describes a metered dose dispensing valve including a movable agitator in the metering chamber. Manual shaking of the valve causes movement of the agitator therein which is stated to assist mixing of the drug suspension in the metering chamber.

PCT Patent Application No. WO96/08284 describes an inhalation device for dispensing powder form medicament including a movable weight which is configured to strike an anvil upon manual shaking of the device. The striking action causes a jolt which acts such as to assist transfer of the powder from a reservoir container to a metering recess formed in a dosing member.

Whilst manual shaking is a generally effective means for providing agitation it is sometimes inconvenient for the patient. In social situations, the patient often wishes to administer their medicament discretely and without drawing attention to themselves. Manual shaking of the dispenser is difficult to perform in a discrete fashion. This can lead to patient embarrassment. There is also always the possibility that the patient forgets to shake the dispenser, or does so inadequately, thereby affecting the medicament dose deliverable.

The Applicants have now developed a medicament dispenser which requires little or no manual shaking. The dispenser comprises a medicament container and an agitator for agitating the contents of the medicament container. A drive is provided for driving the agitator independently of any movement of the container. The dispenser therefore provides for agitation of the medicament container and contents thereof without requiring shaking by the patient.

### Summary of Invention

According to one aspect of the present invention there is provided a medicament dispenser according to claim 1.

Preferably, the movable element is freely movable within the container.

In another aspect, the movable element is mechanically coupled to the driver.

In another aspect, the movable element is pneumatically drivable.

In another aspect, the movable element is hydraulically drivable.

In another aspect, the movable element is electrically drivable.

Preferably, the movable element is shaped to create turbulence within the container when agitated.

In one aspect, the agitator creates regions of pressure difference within the container.

In one aspect, the driver is mounted to the container. The driver may be external to, or internal to, the container and may be permanently or reversibly attached to the container.

In another aspect, the medicament dispenser additionally comprises a housing. Preferably the driver is mounted to the housing. The driver may be external to, or internal to, the housing and may be permanently or reversibly attached to the housing.

The propellant preferably comprises liquefied HFA134a, HFA-227 or carbon dioxide.

The medicament is preferably selected from the group consisting of albuterol, salmeterol, fluticasone propionate, beclomethasone dipropionate, salts or solvates thereof and any mixtures thereof.

### Brief Description of the Invention

The invention will now be described further with reference to the accompanying drawings in which:
Figure 1 is a cut-away perspective view of a first medicament dispenser in accord with the present invention;
Figure 2 is a sectional view of a second medicament dispenser in accord with the present invention;
Figure 3 is a sectional view of a third medicament dispenser in accord with the present invention;
Figures 4a and 4b are sectional views of a fourth medicament dispenser in accord with the present invention;

### Detailed Description of the Invention

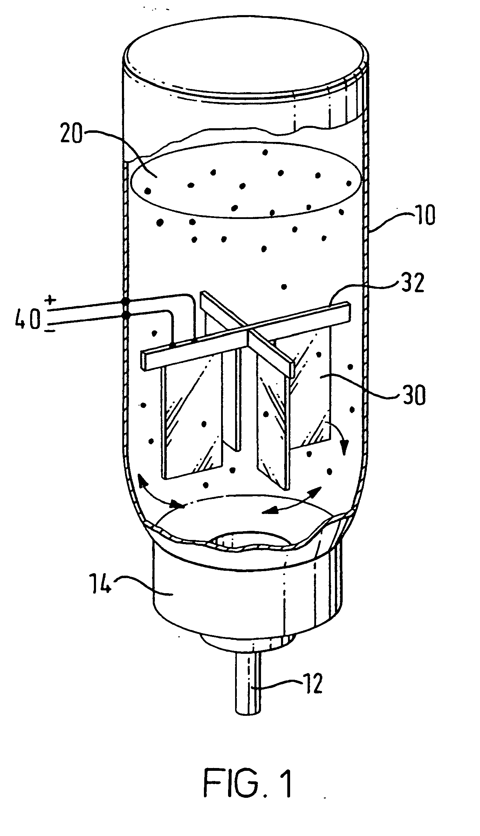

Figure 1. shows a medicament dispenser suitable for use in a metered dose inhaler (MDI) for delivery of inhalable medicament. The dispenser comprises an aerosol can 10 having a dispenser outlet in the form of a metering valve 12. The valve gasket 14 is fixedly attached (typically by crimping) to the aerosol can body 10. The can 10 comprises a suspension 20 of medicament in a propellant.

Within the can 10 there are provided four vibratable flippers 30 mounted on cross-mounting 32. Each vibratable flipper 30 is comprised of a bimetallic strip which flexes on application of electrical current. In alternative embodiments, the flippers may also comprise piezoelectric materials. The cross-mounting 32, and hence each flipper 30, is connected to external electric power source 40 which may be in the form of a battery or a capacitor. On application of electric power the flippers 30 vibrate thereby resulting in agitation of the suspension 20.

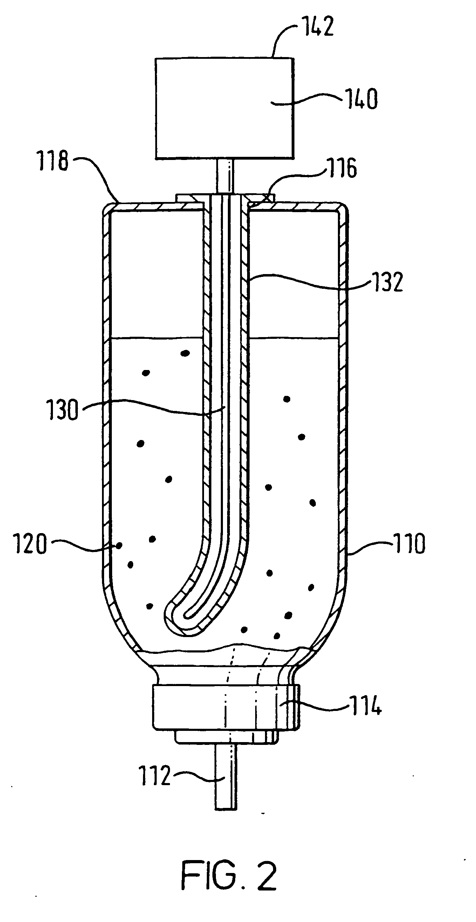

Figure 2. also shows a medicament dispenser suitable for use in a metered dose inhaler (MDI) for delivery of inhalable medicament. The dispenser comprises an aerosol can 110 having a dispenser outlet in the form of a metering valve 112. The valve gasket 114 is fixedly attached (typically by crimping) to the aerosol can body 110. The can 110 comprises a suspension 120 of medicament in a propellant.

It may be seen that an access hole 116 is provided in the base 118 of the aerosol can 110 for receipt of flexible elongate sheath 132. The sheath 132 typically comprises a latex, rubber or elastomeric material and may be seen to protrude well into the interior of the can 110. Within the sheath 132 there is provided drive rod 130 which comprises a resiliently flexible material. The drive rod 130 connects to drive motor 140 which may for example, be an electric or clockwork motor. The drive motor 140 may in embodiments be configured to provide oscillatory or rotatory drive motion to the drive rod 130. On application of the drive energy the drive rod 130 moves within the sheath 132 thereby resulting in agitation of the suspension 120.

In an alternative embodiment, the electric or clockwork drive motor 140 is replaced by a mechanical push-drive which is capable of translating a push motion (e.g. at the top 142 of the drive) into a rotatory drive motion. Such a push-drive could incorporate a suitably configured gear train or worm and helix drive mechanism. In known metered dose inhalers wherein the can 110 sits within an actuator housing, the dispensing action typically requires the user to push down on the base 118 of the can 110 such that the valve 112 is released. It may be appreciated that the use of the push-drive will facilitate a agitation-dispensing sequence in which a first user pushing action on the push-drive results in agitation of the suspension 120 and a second user pushing action (which may be a continuation of the first) results in dispensing of the suspension 120.

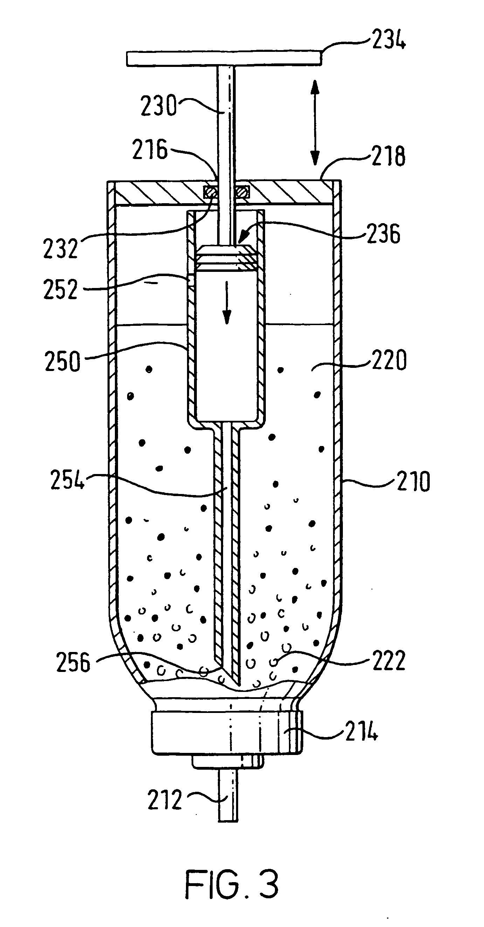

Figure 3. shows a further medicament dispenser suitable for use in a metered dose inhaler (MDI) for delivery of inhalable medicament. The dispenser comprises an aerosol can 210 having a dispenser outlet in the form of a metering valve 212. The valve gasket 214 is fixedly attached (typically by crimping) to the aerosol can body 210. The can 210 comprises a suspension 220 of medicament in a propellant.

It may be seen that an access hole 216 is provided in the base 218 of the aerosol can 210 for receipt of dynamic seal 232. The dynamic seal 232 typically comprises a rigid elastomeric material and may be seen to form a liner to the access hole 216. Protruding through the dynamic seal 232 there is provided piston drive shaft 230. Piston drive shaft 230 has a shaped plunger head 234 which may in embodiments, be connected to a drive motor (not shown) such as an electric or clockwork motor. Piston 236 is received by cylinder 250 within which it is drivable. It may be seen that cylinder 250 includes entry hole 252 and narrows at its lower end to form tube 254 having exit hole 256.

Agitation of the suspension 220 within the can 210 is achieved by a driving movement of the piston 236 within the cylinder 250. In more detail, it can be seen that when the piston 236 is in the withdrawn position (as shown in Figure 3) gas may enter cylinder 250 through entry hole 252. As the piston 236 is moved downwards the gas is initially trapped in the cylinder 250, then pushed through tube 254 and finally expelled through exit hole 256. Expulsion of the gas results in formation of bubbles 222 and flow agitation in the suspension 220.

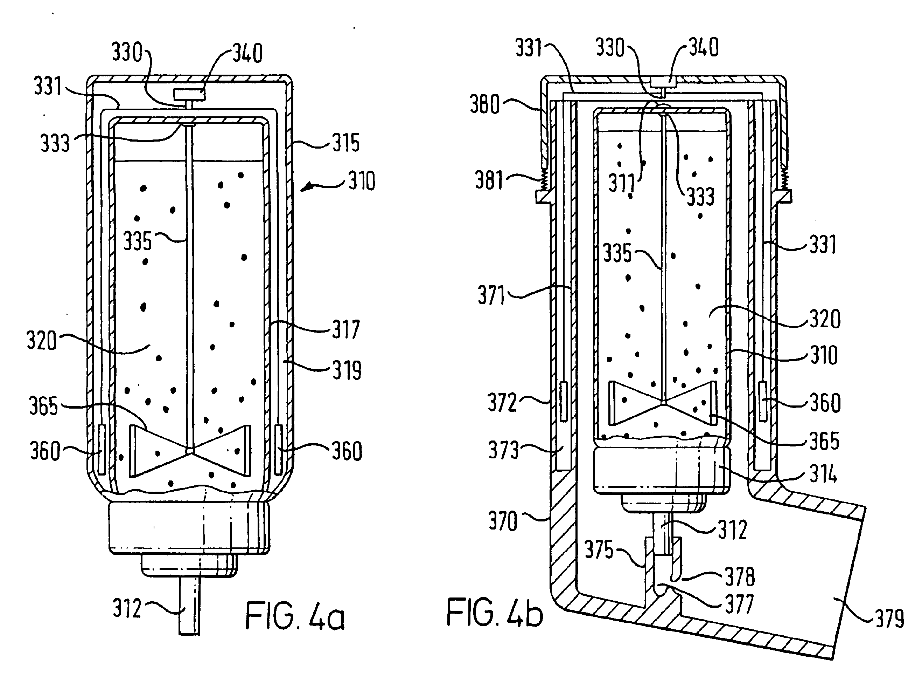

Figure 4a shows a medicament dispenser suitable for use in a metered dose inhaler for delivery of inhalable medicament. The dispenser comprises an aerosol canister 310 having a dispenser outlet 312 in the form of a metering valve. The canister 310 has an external 315 and internal 317 wall with cavity 319 therebetween. A suspension 320 of a medicament in a propellant is contained within internal wall 317 of the canister. A magnetic impeller 365 is located within canister 310 and is free to rotate around axis 333 on shaft 335.

Within cavity 319 there is provided an electric or clockwork motor 340 which connects via drive shaft 330 to magnet 360. The electric motor may be powered by any suitable means, such as a battery (not shown). The magnet 360 may be rotated or oscillated on arm 331, connecting to drive shaft 330, around internal wall 317 when powered by the motor 340. It may be appreciated that rotation or oscillation of arm 331 and of magnet 360 will result in rotation or oscillation of magnetic impeller 365.

Agitation of the suspension 320 within the aerosol canister 310 is achievable by the following steps. The motor 340 is actuated by means of a pressure switch (not shown) to rotate drive shaft 330 and arm 331, thereby rotating magnet 360 within cavity 319. The magnet 360 on arm 331 acts magnetically on the magnetic impeller 365 within the can 310 such that the impeller 365 is also rotated. Rotation of the magnetic impeller 365 agitates the suspension 320.

Figure 4b shows a variation of the dispenser of Figure 4a. In the metered dose inhalation device of Figure 4b an aerosol canister 310 is located within housing 370 such that dispenser outlet 312 is positioned within passage 377 of support 375. A second passage 378 is provided within support 375 and is directed towards the interior of outlet 379.

Aerosol canister 310 contains a suspension 320 of a medicament in a propellant. Magnetic impeller 365 is located within canister 310 and is free to rotate on shaft 335 about axis 333. Detachable cap 380 encloses aerosol canister 310 within housing 370. The cylindrical housing 370 divides into an internal 371 and external 372 wall with cavity 373 therebetween. In the position shown, magnet 360 is suspended from arm 331, connected to drive shaft 330, and is free to rotate or oscillate within the cavity 373 when powered by electric or clockwise motor 340. It will be appreciated that rotation of magnet 360 leads to rotation of magnetic impeller 365 and thus agitation of suspension 320.

To use the device, cap 380 is compressed thereby activating a pressure switch (not shown) of motor 340 leading to rotation of magnet 360 within cavity 373. Magnetic impeller 365 is thus rotated within canister 310 and agitates suspension 320. Further compression of cap 380 pushes drive shaft 330 against canister block 311, stopping rotation of the drive shaft and moving canister 310 relative to valve member 312. This action opens the valve and a dose of medicament contained within the aerosol canister is dispensed through passage 378 into outlet 379 to be inhaled by the patient. On removal of the pressure, resilient material 381 returns cap 380 to its resting position.

It may be appreciated that any of the parts of the dispenser which contact the medicament suspension may be coated with materials such as fluoropolymer materials which reduce the tendency of medicament to adhere thereto. Any movable parts may also have coatings applied thereto which enhance their desired movement characteristics. Frictional coatings may therefore be applied to enhance frictional contact and lubricants used to reduce frictional contact as necessary.

The medicament dispenser of the invention is suitable for dispensing medicament, particularly for the treatment of respiratory disorders such as asthma and chronic obstructive pulmonary disease. Appropriate medicaments may thus be selected from, for example, analgesics, e.g., codeine, dihydromorphine, ergotamine, fentanyl or morphine; anginal preparations, e.g., diltiazem; antiallergics, e.g., cromoglycate (e.g. s the sodium salt), ketotifen or nedocromil (e.g. as the sodium salt); antiinfectives e.g., cephalosporins, penicillins, streptomycin, sulphonamides, tetracyclines and pentamidine; antihistamines, e.g., methapyrilene; anti- inflammatories, e.g., beclomethasone (e.g. as the dipropionate ester), fluticasone (e.g. as the propionate ester), flunisolide, budesonide, rofleponide, mometasone e.g. as the furoate ester), ciclesonide, triamcinolone (e.g. as the acetonide) or 6α, 9α-difluoro-11β-hydroxy-16α-methyl-3-oxo-17α-propionyloxy-androsta-1,4-diene-17β-carbothioic acid S-(2-oxo-tetrahydro-furan-3-yl) ester; antitussives, e.g., noscapine; bronchodilators, e.g., albuterol (e.g. as free base or sulphate), salmeterol (e.g. as xinafoate), ephedrine, adrenaline, fenoterol (e.g. as hydrobromide), formoterol (e.g. as fumarate), isoprenaline, metaproterenol, phenylephrine, phenylpropanolamine, pirbuterol (e.g. as acetate), reproterol (e.g. as hydrochloride), rimiterol, terbutaline (e.g. as sulphate), isoetharine, tulobuterol or 4-hydroxy-7-[2-[[2-[[3-(2-phenylethoxy)propyl]sulfonyl]ethyl]amino]ethyl-2(3H)-benzothiazolone; adenosine 2a agonists, e.g. 2R,3R,4S,5R)-2-[6-Amino-2-(1S-hydroxymethyl-2-phenyl-ethylamino)-purin-9-yl]-5-(2-ethyl-2H-tetrazol-5-yl)-tetrahydro-furan-3,4-diol (e.g. as maleate); α4 integrin inhibitors e.g. (2S)-3-[4-({[4-(aminocarbonyl)-1-piperidinyl] carbonyl}oxy)phenyl]-2-[((2S)-4-methyl-2-{[2-(2-methylphenoxy) acetyl]amino}pentanoyl)amino] propanoic acid (e.g. as free acid or potassium salt), diuretics, e.g., amiloride; anticholinergics, e.g., ipratropium (e.g. as bromide), tiotropium, atropine or oxitropium; hormones, e.g., cortisone, hydrocortisone or prednisolone; xanthines, e.g., aminophylline, choline theophyllinate, lysine theophyllinate or theophylline; therapeutic proteins and peptides, e.g., insulin or glucagon; vaccines, diagnostics and gene therapies. It will be clear to a person skilled in the art that, where appropriate, the medicaments may be used in the form of salts, (e.g., as alkali metal or amine salts or as acid addition salts) or as esters (e.g., lower alkyl esters) or as solvates (e.g., hydrates) to optimise the activity and/or stability of the medicament and/or to minimise the solubility of the medicament in the propellant. Preferred medicaments are selected from albuterol, salmeterol, fluticasone propionate and beclomethasone dipropionate and salts or solvates thereof, e.g., the sulphate of albuterol and the xinafoate of salmeterol.

Medicaments can also be delivered in combinations. Preferred formulations containing combinations of active ingredients contain salbutamol (e.g., as the free base or the sulphate salt) or salmeterol (e.g., as the xinafoate salt) or formoterol (e.g. as the fumarate salt) in combination with an antiinflammatory steroid such as a beclomethasone ester (e.g., the dipropionate) or a fluticasone ester (e.g., the propionate) or budesonide. A particularly preferred combination is a combination of fluticasone propionate and salmeterol, or a salt thereof (particularly the xinafoate salt). A further combination of particular interest is budesonide and formoterol (e.g. as the fumarate salt).

It will be understood that the present disclosure is for the purpose of illustration only and the invention extends to modifications, variations and improvements thereto.

## Claims

1. A medicament dispenser for use in a metered dose inhaler comprising:
an aerosol container (10;110;210;310) having a dispensing outlet and a metering valve (12;112;212;312) at the dispensing outlet, the container containing a suspension (20;120;220;320) of a medicament In a propellant; and
an agitator for agitating the contents of the container, the agitator comprising a movable element (30;130;132;236; 335,365) within said container. **characterised by** the provision of:
a driver (32,40;140;234;340,360) for driving said agitator independently of any movement of the container.

2. A medicament dispenser according to claim 1, wherein the movable element is freely movable within the container.

3. A medicament dispenser according to claim 2, wherein the movable element (30;130,132;236) is mechanically coupled to the driver (32.40;140;234).

4. A medicament dispenser according to claim 1, wherein the movable element is pneumatically drivable,

5. A medicament dispenser according to claim 1, wherein the movable element is hydraulically drivable.

6. A medicament dispenser according to claim 1, wherein the movable element (30;130,132;236) is electrically drivable.

7. A medicament dispenser according to any of claims 1 to 6, wherein the movable element is shaped to create turbulence within the container when agitated.

8. A medicament dispenser according to claim 1, wherein the agitator creates regions of pressure difference within the container.

9. A medicament dispenser according to any of claims 1 to 8, wherein the driver (32,40;140;234;340,360) is mounted to the container.

10. A medicament dispenser according to claim 1 or 2, additionally comprising a housing (370,380).

11. A medicament dispenser according to claim 10, wherein the driver (340,360) is mounted to said housing (370,380).

12. A medicament dispenser according to any preceding claim, wherein said propellant comprises liquefied HFA134a, HFA-227 or carbon dioxide.

13. A medicament dispenser according to any preceding claim, wherein the medicament is selected from the group consisting of albuterol, salmeterol, fluticasone propionate, beclomethasone dipropionate, salts or solvates thereof and any mixtures thereof.

## Patentansprüche

1. Medikamentenspender zur Verwendung in einem Inhalator für abgemessene Dosierungen, umfassend:
einen Aerosolbehälter (10, 110, 210, 310) mit einem Spenderauslass und einem Abmessventil (12, 112, 212, 312) am Spenderauslass, wobei der Behälter eine Suspension (20, 120, 220, 320) eines Medikaments in einem Treibmittel aufnimmt, und
einem Mischer zum Mischen der Inhalte des Behälters, wobei der Mischer ein bewegliches Element (30, 130, 132, 236 335, 365) innerhalb des Behälters umfasst, **gekennzeichnet durch** die Bereitstellung von: einen Antreiber (32, 40, 140, 234, 340, 360) zum Antreiben des Mischers unabhängig von jeglicher Bewegung des Behälters.

2. Medikamentenspender gemäß Anspruch 1, bei dem das bewegliche Element innerhalb des Behälters frei beweglich ist.

3. Medikamentenspender gemäß Anspruch 2, bei dem das bewegliche Element (30, 130, 132, 236) mit dem Antreiber (32, 40, 140, 234) mechanisch gekoppelt ist.

4. Medikamentenspender gemäß Anspruch 1, bei dem das bewegliche Element pneumatisch antreibbar ist.

5. Medikamentenspender gemäß Anspruch 1, bei dem das bewegliche Element hydraulisch antreibbar ist.

## Revendications

1. Distributeur de médicament à utiliser dans un inhalateur à dose mesurée comprenant :
un récipient d'aérosol (10 ; 110 ; 210 ; 310) doté d'une sortie de distribution et d'une soupape de mesure (12 ; 112 ; 212 ; 312) à la sortie de distribution, le récipient contenant une suspension (20 ; 120 ; 220 ; 320) d'un médicament dans un propulseur, et
d'un agitateur pour agiter le contenu du récipient, l'agitateur comprenant un élément mobile (30 ; 130 ; 132 ; 236 ; 335 ; 365) dans ledit récipient ; **caractérisé par** la fourniture
d'un système d'entraînement (32, 40 ; 140 ; 234 ; 340 ; 360) pour entraîner ledit agitateur indépendamment de tout mouvement du récipient.

2. Distributeur de médicament selon la revendication 1, dans lequel l'élément mobile est librement mobile dans le récipient.

3. Distributeur de médicament selon la revendication 2, dans lequel l'élément mobile (30 ; 130 ; 132 ; 236) est mécaniquement relié au système d'entraînement (32, 40 ; 140 ; 234).
